# EUROPÄISCHE PATENTSCHRIFT

(11) **EP 1 446 079 B1**
(45) Veröffentlichungstag und Bekanntmachung des Hinweises auf die Patenterteilung: **25.07.2007**
(21) Anmeldenummer: 02781552.1
(22) Anmeldetag: 21.11.2002
(51) Int. Cl.: A61F 9/007

(54) **VORRICHTUNG ZUR DURCHFÜHRUNG OPHTALMOLOGISCHER EINGRIFFE**
DEVICE FOR PERFORMING OPHTHALMOLOGICAL OPERATIONS
DISPOSITIF PERMETTANT D'EFFECTUER DES INTERVENTIONS OPHTALMOLOGIQUES

(30) Priorität: 22.11.2001 WO PCT/IB01/02224
(43) Veröffentlichungstag der Anmeldung: 18.08.2004
(60) Teilanmeldung: 07007700.3
(73) Patentinhaber: Haefliger, Eduard Anton, 4057 Basel (CH)
(72) Erfinder: Haefliger, Eduard Anton, 4057 Basel (CH)
(74) Vertreter: Blum, Rudolf Emil
(86) Internationale Anmeldenummer: PCT/IB2002/004845
(87) Internationale Veröffentlichungsnummer: WO 2003/043549

(56) Entgegenhaltungen:
- EP-A- 0 858 788
- WO-A-01/37767
- WO-A-01/74427
- WO-A-93/20784
- DE-A- 19 842 799
- DE-C- 3 416 490
- FR-A- 2 695 821
- SU-A- 938 994

## Beschreibung

### Hintergrund

Die Erfindung betrifft eine Vorrichtung zur Behandlung von grünem Star durch Erzeugen von Öffnungen zwischen der Vorderkammer und dem Schlemmschen Kanal eines Auges gemäss Oberbegriff der unabhängigen Ansprüche.

WO 01/37767 beschreibt verschiedene Vorrichtungen zur Behandlung von grünem Star. Diese Vorrichtungen sind mit einer Sonde versehen, die in die Vorderkammer des Auges eingeführt werden kann um Öffnungen im Trenngewebe zwischen der Vorderkammer und dem Schlemmschen Kanal zu erzeugen. In einer Ausführung besitzt diese Sonde einen Kanal, welcher z.B. mit einer Aspirations-und Irrigationseinheit in Verbindung stehen kann. Neben diesem Kanal kann ein Schneid-, Greif oder Klemmwerkzeug angeordnet sein um eine Öffnung im Trenngewebe zu erzeugen.

Eine derartige Vorrichtung vermag jedoch nicht zu garantieren, dass nur das Trenngewebe zwischen dem Schlemmschen Kanal und der Vorderkammer zerstört wird. Selbst bei sehr sorgfältiger Handhabung kann z.B. auch Gewebe hinter dem Schlemmschen Kanal beschädigt werden, d.h. Gewebe, welches auf der von der Vorderkammer abgewandten Seite des Schlemmschen Kanals liegt. Ferner kann abgeschnittenes Gewebe in den Schlemmschen Kanal gelangen.

### Darstellung der Erfindung

Es stellt sich deshalb die Aufgabe, eine Vorrichtung bereitzustellen, welche eine möglichst zuverlässige Erstellung von Öffnungen im Trenngewebe zum Schlemmschen Kanal erlaubt.

Diese Aufgabe wird vom unabhängigen Anspruch gelöst.

Die Erfindung basiert auf dem Gedanken, dass der Saugkanal in der Sonde dazu verwendet werden kann, das Trenngewebe zum Abtrennen anzusaugen und sodann das angesogene Gewebe abzutrennen. Dies wird dadurch erreicht, dass das Trennmittel direkt im Saugkanal angeordnet wird, was ein gezieltes Abtrennen im Bereich des Saugkanals erlaubt. In einem bevorzugten Aspekt ist das Trennmittel ausgestaltet, um das Trenngewebe entlang einer Linie abzutrennen, die sich im wesentlichen um eine Längsachse des Saugkanals herum erstreckt. Auch in diesem Fall kann ein gezieltes Abtrennen im Bereich des Saugkanals erzielt werden. Auf diese Weise ist es möglich, das abgetrennte Gewebe durch den Saugkanal abzusaugen. Ausserdem unterstützt die Saugwirkung den Trennprozess.

Das Trennmittel ist als Schneidewerkzeug ausgestaltet, mit welchem das Trenngewebe mechanisch durchschnitten werden kann.

Anspruchsgemäss wird die Trenn- bzw. Schneidekante des Trennmittels stationär im Saugkanal maximal bis zu dessen Mündung angeordnet. Dabei wird sie so positioniert, dass durch Unterdruck im Saugkanal Trenngewebe zum Schneiden gegen die Trennkante gezogen werden kann. Bei dieser Ausführung kann auf eine mechanische Bewegung des Trennmittels unter Umständen verzichtet werden.

Vorzugsweise ist ein Schneidewerkzeug im Saugkanal vorgesehen, mit einer Schneidekante, die maximal bis zu dessen Mündung reicht. Dies hat den Vorteil, dass die Schneidkante ihre Wirkung nur bei Gewebe entfaltet, welches in den Saugkanal eingesogen wird. Eine versehentliche Verletzung von Gewebe kann somit verhindert werden. Insbesondere wird beim Schneiden das hinter dem Schlemmschen Kanal liegende Gewebe nicht verletzt.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine Darstellung der erfindungsgemässen Vorrichtung bei einer Behandlung von grünem Star anhand einer Schnittfigur durch die Vorderkammer eines Auges,
Fig. 2 einen Längsschnitt einer ersten nicht beanspruchten Ausführung der Vorrichtung,
Fig. 3 die Ausführung nach Fig. 2 mit Schneidewerkzeug in der Abtrennstellung,
Fig. 4 einen Längsschnitt einer zweiten Ausführung der Erfindung,
Fig. 5 einen Längsschnitt einer dritten Ausführung der Erfindung,
Fig. 6 die Ausführung nach Fig. 5 mit Schneidewerkzeug in der Abtrennstellung (nicht beansprucht),
Fig. 7 einen Längsschnitt einer vierten Ausführung der Erfindung,
Fig. 8 einen Längsschnitt einer fünften Ausführung der Vorrichtung,
Fig. 9 einen Längsschnitt einer sechsten Ausführung der Vorrichtung und
Fig. 10 einen Längsschnitt durch den proximalen Abschnitt einer Ausführung der Erfindung mit piezoelektrischem Antrieb.

### Wege zur Ausführung der Erfindung

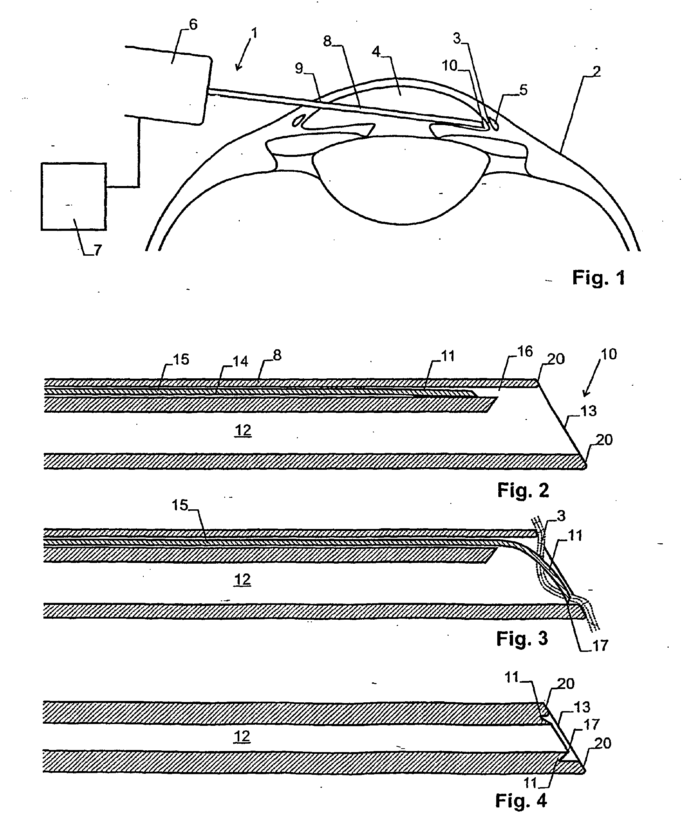

In Fig. 1 wird eine Vorrichtung 1 gezeigt, welche zur Durchführung mikrochirurgischer Eingriffe in einem Auge 2 ausgestaltet ist. Insbesondere ist die Vorrichtung dazu geeignet, zusätzliche Öffnungen im Trenngewebe 3 zwischen der Vorderkammer 4 und dem Schlemmschen Kanal 5 zu schaffen. Derartige Öffnungen verbessern den Abfluss von Flüssigkeit aus der Vorderkammer 4 und werden erzeugt, um einen Überdruck im Auge 1 zu reduzieren und den grünen Star zu behandeln.

Die Vorrichtung 1 umfasst einen Handgriff 6, eine schematisch dargestellte Absaugvorrichtung 7 und eine Sonde 8. Die Sonde 8 besitzt eine Länge, Dicke und Form, die es erlauben, sie z.B. durch einen chirurgisch vorbereiteten Schlitz 9 in die Vorderkammer 4 einzuführen, und zwar so, dass ihr distales Ende 10 zum Trenngewebe 3 zwischen Vorderkammer 4 und Schlemmschen Kanal 5 geführt werden kann.

Die Sonde 8 besitzt im wesentlichen die Form einer Nadel. Sie kann gerade oder gebogen sein.

Fig. 2 und 3 zeigen einen Längsschnitt durch eine nicht beanspruchte Ausführung der Sonde 8 im Bereich des Endes 10. Wie ersichtlich, besitzt die Sonde 8 einen Saugkanal 12, der sich von einer Mündung 13 am Ende 10 durch die ganze Sonde 8 in den Griff 6 erstreckt und mit der Absaugvorrichtung 7 verbunden ist. Durch Einschalten der Absaugvorrichtung kann in Saugkanal 12 somit ein Unterdruck erzeugt werden.

Wie aus den Figuren ersichtlich, ist Mündung 13 stirnseitig am distalen Ende der Sonde 8 angeordnet. Dies erlaubt es, die Mündung in einfacher Weise bis an die Trennwand des Schlemm'schen Kanals zu führen. Wie ebenfalls aus den Figuren ersichtlich, liegt Mündung 13 vorzugsweise nicht senkrecht sondern Schräg zur Längsachse der Sonde. Auch diese Massnahme vereinfacht es, die Mündung an die Trennwand des Schlemm'schen Kanals zu führen, wie dies insbesondere aus Fig. 1 ersichtlich ist.

In der in Fig. 2 und 3 gezeigten Vorrichtung erstreckt sich ein weiterer Kanal 14 entlang der Sonde 8, in welchem längsverschiebbar eine Trenn- bzw. Schneidevorrichtung 15 angeordnet ist. Fig. 2 zeigt die Trenn-bzw. Schneidevorrichtung 15 in der Ruhestellung, Fig. 3 in der Abtrennstellung. Zum Verschieben der Trenn- bzw. Schneidevorrichtung 15 zwischen Ruhestellung und Abtrennstellung kann z.B. ein entsprechendes Betätigungsorgan im Griff 6 vorgesehen sein (nicht gezeigt).

Das distale Ende der Trenn-bzw. Schneidevorrichtung, welches das eigentliche Trennmittel bzw. Schneidewerkzeug 11 bildet, ist gegen den Saugkanal 12 hin vorgebogen. Wird die Trenn- bzw. Schneidevorrichtung 15 aus der Ruhestellung in die Abtrennstellung gebracht, tritt das Schneidewerkzeug 11 durch eine zwischen dem Saugkanal 12 und dem weiterem Kanal 14 vorgesehene Öffnung 16 in den Saugkanal 12 in einem Bereich der Mündung 13. Dabei bewegt es sich über den Querschnitt des Saugkanals 12, durchtrennt mit einer Schneidekante 17 am Ende 10 in die Mündung eingesogenes Trenngewebe 3 und erzeugt somit die gewünschte Öffnung. Der abgetrennte Teil des Trenngewebes 3 kann durch den Saugkanal 12 abgesogen werden.

Eine zweite Ausführung der erfindungsgemässen Vorrichtung ist in Fig. 4 dargestellt. Hier wird das Trennmittel bzw. Schneidwerkzeug 11 von einer kreisförmigen bzw. ovalen Schneidekante 17 erzeugt, die sich entlang der Innenwand des Saugkanals 12 erstreckt. Die Schneidekante 17 ist knapp hinter oder an der Mündung 13 angeordnet. Wird Trenngewebe 3 in die Mündung 13 eingesogen, so trifft es auf die Schneidekante 17 und wird abgetrennt.

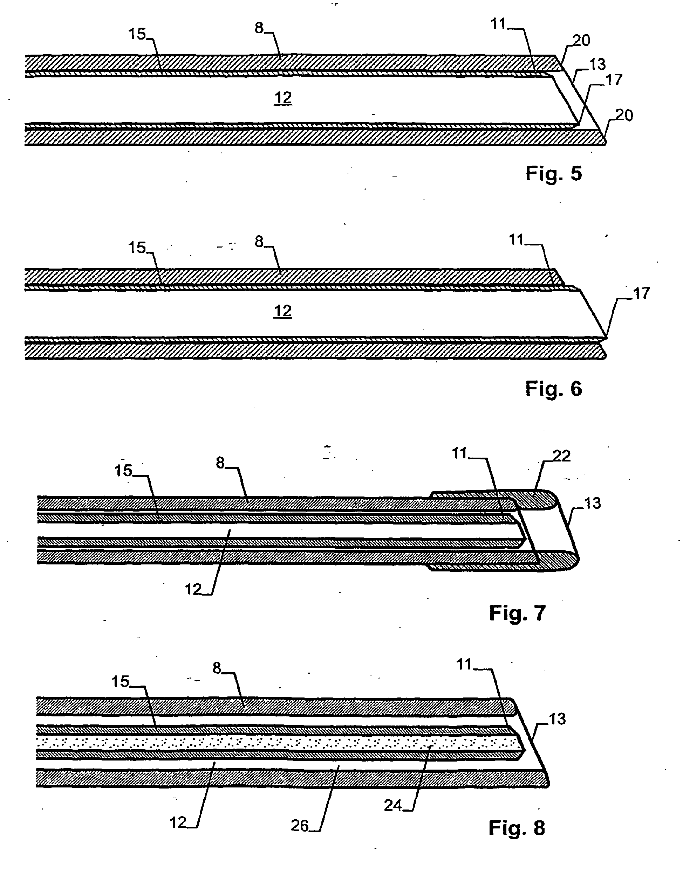

Eine dritte Ausführung der erfindungsgemässen Vorrichtung ist in Fig. 5 und 6 dargestellt. Hier wird die Schneidevorrichtung 15 von einem Rohrstück gebildet, das im Saugkanal 12 angeordnet ist. Das distale Ende des Rohrs bildet das Schneidewerkzeug 11 mit Schneidekante 17.

Vorzugsweise (nicht beansprucht) ist das Schneidevorrichtung 15 bzw. Schneidewerkzeug 11 längsverschiebbar und kann von der in Fig. 5 gezeigten Ruhestellung in die in Fig. 6 gezeigte Abtrennstellung bewegt werden. In der Ruhestellung befindet sich die Schneidekante 17 hinter der Mündung 13, in der Abtrennstellung ragt die Schneidekante 17 um eine gegebene Distanz über die Mündung 13 hinaus. Die Distanz entspricht ungefähr einer typischen Dicke des Trenngewebes 3. Auf diese Weise kann Trenngewebe, das von der Mündung 13 angesogen wird, abgetrennt werden.

Es ist auch denkbar (nicht beansprucht), die Schneidevorrichtung 15 drehbar im ihre Längsachse auszugestalten, so dass sie zumindest über einen beschränkten Winkelbereich gegenüber der Sonde 8 gedreht werden kann. Dies erlaubt es, durch Hin- und Herdrehen der Schneidevorrichtung eine zusätzliche Schneidewirkung zu erzielen.

In der hier gezeigten zweiten und dritten Ausführung erstreckt sich die Schneidekante 17 um den Umfang des Saugkanals 12 an dessen Innenseite entlang. Es ist auch denkbar, dass die Schneidekante 17 in mehrere, um die Längsachse des Saugkanals 12 angeordnete Abschnitte unterteilt ist und nicht eine durchgehende Kante bildet. Vorzugsweise trennt das Schneidewerkzeug das Trenngewebe 3 entlang einer im wesentlichen kreisförmigen Schnittlinie ab.

Wie aus Fig. 2 bis 6 ersichtlich, sind die Kanten der Mündung 13 vorzugsweise stumpf oder abgestumpft, in Gegensatz zu der vorzugsweise unter spitzem Winkel zulaufenden Schneidekante 17. Ausserdem ist die Mündung 13 von einer im wesentlichen flachen Schulterfläche 20 umgeben, auf die das Trenngewebe abstützen kann. Dadurch wird erreicht, dass das Trenngewebe 3 gezielt durch die Wirkung der Schneidekante 17 abgetrennt wird. Es ist jedoch auch möglich, die Schneidekante 17 so anzuordnen, dass sie die Randkante der Mündung 13 bildet.

Der Saugkanal 12 kann runden oder eckigen Querschnitt haben. Insbesondere in der ersten Ausführung nach Fig. 2 und 3 ist er zumindest im Bereich des distalen Endes 10 eher eckig, so dass er vom Schneidewerkzeug 11 vollständig überstrichen werden kann, während er in den Ausführungen nach Fig. 4 bis 6 vorzugsweise ungefähr rund oder elliptisch ist.

In einer weiteren Ausführung ist, wie in Fig. 7 dargestellt, am distalen Ende der Sonde 8 als Abstützvorrichtung eine Manschette 22 angeordnet. Sie besteht aus deformierbarerem Material als die Sonde selbst, beispielsweise aus Silikon, und erlaubt eine bessere Abdichtung des Saugkanals 12 gegen das Gewebe.

In der Anwendung der hier beschriebenen Vorrichtung wird das distale Ende 10 der Sonde 8 durch die Vorderkammer 4 des Auges zum Trenngewebe 3 geführt, wobei in dieser Phase das Schneidewerkzeug 11 der Ausführungen gemäss Fig. 2 und 3 bzw. 5 und 6 in der Ruhestellung ist. Sodann wird ein Stück Trenngewebe in die Mündung 13 des Saugkanals 12 eingesogen. In der Ausführung nach Fig. 4 wird es dabei automatisch von der Schneidekante 17 durchgetrennt. In-der Ausführung nach Fig. 2 und 3 bzw. 5 und 6 wird die Schneidevorrichtung 15 in die Abtrennstellung gebracht, so dass das eingesogene Trenngewebe abgetrennt wird. Falls es sich zeigt, dass die Abtrennung nicht vollständig ist, so kann das verbleibende Gewebestück mit einer geeigneten Pincette entfernt werden.

Während die Sonde 8 durch die Vorderkammer 4 des Auges zum Trenngewebe 3 geführt wird, arbeitet die Absaugvorrichtung mit relativ geringer Leistung oder sie ist abgeschaltet. Sobald die Sonde in Saugkontakt mit dem Trenngewebe 3 ist, wird vorzugsweise ein kurzer "Saugpuls" mit höherer Saugleistung erzeugt, während dem das Trenngewebe abgetrennt werden kann. Wenn die Absaugvorrichtung 7 während dem Einführen mit geringer Saugleistung betrieben wird, so kann aufgrund des Druckabfalls und/oder des Rückgangs der Absaugrate festgestellt werden, wann sich die Sonde 8 in Saugkontakt mit dem Trenngewebe 3 befindet.

Vorzugsweise ist die Absaugvorrichtung deshalb ausgestaltet zur Erzeugung kurzer Saugpulse von z.B. 50 - 200 ms. Für einen Betrieb mit geringer Saugleistung während dem Einführen muss sie deshalb ausgestaltet sein um kurze Saugpulse einer ersten, höheren Saugleistung und ein dauerendes Saugen bei einer zweiten, tieferen Saugleistung zu erlauben. Die Ausgestaltung einer entsprechenden Pumpe mit Pumpensteuerung und entsprechenden Bedienelementen zum Auslösen des Saugpulses liegen im Können des normalen Fachmanns.

Wird, wie in den Ausführungen gemäss Fig. 2 - 4, das Trenn- bzw. Schneidewerkzeug 11, genauer gesagt die Schneidekante 17, im Saugkanal 12 angeordnet und reicht es maximal bis zur Mündung 13, so kann nur Gewebe abgetrennt werden, das in den Saugkanal 12 eingesaugt wird. Dadurch kann eine unbeabsichtigte Verletzung von Gewebe, z.B. an der Aussenseite des Schlemmschen Kanals 5, verhindert werden. In der Ausführung nach Fig. 6 wird eine unbeabsichtigte Verletzung dadurch verhindert, dassdie Schneidekante 17 nur um eine geringe, mit der Dicke des Trenngewebes 3 vergleichbare Distanz über die Mündung 13 hinausgeschoben werden kann.

In den bisher gezeigten Vorrichtungen wurde das Trennmittel 11 von einem Schneidewerkeug gebildet, welches das Trenngewebe durch einen rein mechanischen Schnitt abtrennt. Es ist jedoch auch möglich, das angesaugte Trenngewebe in anderer Weise abzutrennen oder den Schneidvorgang mit anderen Mitteln zu unterstützen.

Fig. 8 zeigt eine Ausführung, bei welcher die Trenn- bzw. Schneidevorrichtung 15 wiederum von einem Rohr in der Sonde 8 gebildet wird, wobei in dieser Ausführung im Innern der Trenn- bzw. Schneidevorrichtung 15 eine Ätzflüssigkeit 24 (z.B. eine Säure oder Lauge) eingefüllt ist. Der Saugkanal 12 liegt in dieser Ausführung zwischen der Trenn- bzw. Schneidevorrichtung 15 und der Wand der Sonde 8.

Auch hier wird ein Stück Trenngewebe in die Mündung 13 des Saugkanals 12 eingesogen. Nun wird mit einer Mikropumpe im Griff 6 eine kleine Menge der Ätzflüssigkeit 24 gegen das distale Ende gepumpt, wo sie mit dem Trenngewebe 3 in Kontakt kommt und dieses mindestens teilweise auflöst und abtrennt. Bevor Ätzflüssigkeit in weitere Teile des Auges austreten kann, wird sie durch den Saugkanal 12 abgesogen.

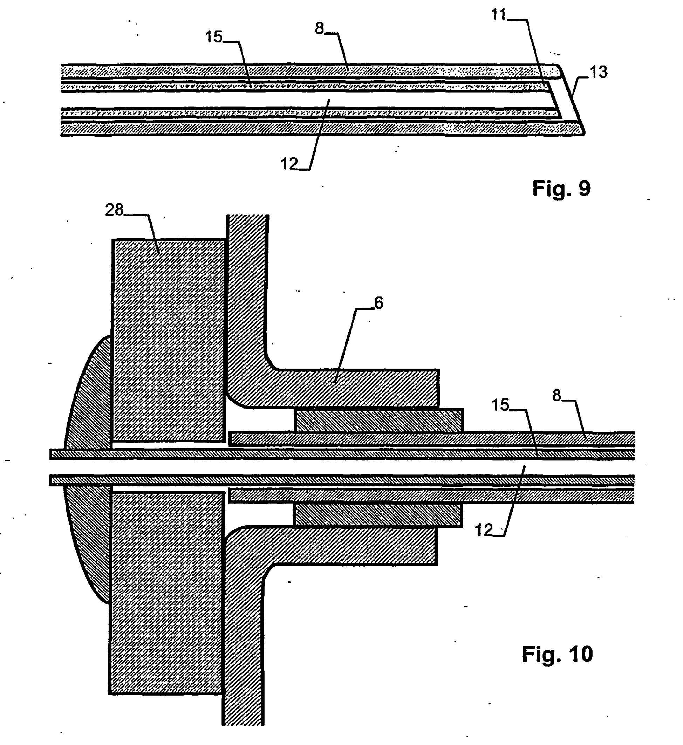

In der Ausführung nach Fig. 9 wird die Trenn-bzw. Schneidevorrichtung 15 von einem rohrförmigen optischen Wellenleiter gebildet, in dessen Mitte der Saugkanal 12 verläuft. Auch hier wird wieder Trenngewebe in die Mündung 13 eingesogen. Sodann wird durch den Wellenleiter ein optischer Lichtpuls hoher Intensität geschickt, der am distalen Ende des Wellenleiters austritt und dort lokal das Trenngewebe abtrennt.

Anstelle eines rohrförmigen optischen Wellenleiters kann auch ein normaler Wellenleiter in Form einer Fiber verwendet werden, der z.B. im von der Sonde gebildeten Saugkanal 12 verläuft.

Die hier gezeigten optischen, chemischen und mechanischen Trennverfahren können auch kombiniert werden.

In allen Fällen kann es auch sinnvoll sein, das Trennmittel 11 und/oder die Sonde 8 in Vibration zu versetzen, insbesondere mit Frequenzen im Ultraschallbereich. Durch eine Vibration des Trennmittels 11 wird der Abtrennvorgang unterstützt. Durch eine Vibration des Trennmittels 11 und/oder der Sonde 8 kann zudem einem Verstopfen des Saugkanals 12 durch eingesogenes Gewebe entgegengewirkt werden.

Fig. 10 zeigt eine Ausführung der Vorrichtung als Schnitt durch das proximale Ende der Sonde 8, bei welcher die Trenn- bzw. Schneidevorrichtung 15 in Vibrationen versetzt werden kann. Hierzu ist ein piezoelektrischer Transducer 28 vorgesehen, welcher auf einer Seite das proximale Ende der Trenn- bzw. Schneidevorrichtung 15 hält und auf der anderen Seite z.B. am Gehäuse des Griffs 6.angeordnet ist. Der Schaft 8 kann ebenfalls am Gehäuse des Griffs 6 abgestützt sein. Durch Beaufschlagen des Transducers 28 mit einer Wechselspannung kann die Trenn-bzw. Schneidevorrichtung 15 in Vibrationen versetzt werden.

In den gezeigten Ausführungen beträgt der Durchmesser der Mündung 13 vorzugsweise zwischen 50 und 200 µm. Da der Schlemm'sche Kanal in der Regel nicht grö-sser als 200 µm ist, sind grössere Durchmesser nachteilig.

Bei der Herstellung des hier beschriebenen Geräts können konventionelle mechanische Bearbeitungsschritte verwendet werden. Insbesondere das distale Ende des Trennmittels 11 kann jedoch auch mittels anisotroper Ätztechnik z.B. aus Silizium geformt werden.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Vorrichtung zur Behandlung von grünem Star durch Erzeugen von Öffnungen zwischen der Vorderkammer und dem Schlemmschen Kanal eines Auges, mit einer Sonde (8), welche geeignet ist zum Einführen in die Vorderkammer derart, dass ein distales Ende (10) der Sonde (8) zu einem Trenngewebe zwischen Vorderkammer und Schlemmschen Kanal führbar ist, wobei am Ende der Sonde (8) ein Schneidewerkzeug (11) zum Auftrennen des Trenngewebes angeordnet ist, und wobei sich durch die Sonde (8) ein Saugkanal (12) erstreckt, der in einer Mündung (13) stirnseitig am distalen Ende der Sonde (8) mündet, **dadurch gekennzeichnet, dass** das Schneidewerkzeug (11) im Saugkanal (12) bei der Mündung (13) angeordnet ist, derart, dass mit dem Schneidewerkzeug (11) in den Saugkanal (12) eingesaugtes Trenngewebe abtrennbar ist, wobei eine Schneidekante (17) des Schneidewerkzeugs (11) stationär im Saugkanal (12) maximal bis zur Mündung (13) angeordnet ist, und wobei die Schneidekante (17) so positioniert ist, dass durch Unterdruck im Saugkanal (12) Trenngewebe zum Schneiden gegen die Schneidekante (17) ziehbar ist.

2. Vorrichtung nach Anspruch 1, **dadurch gekennzeichnet, dass** das Schneidewerkzeug (11) ausgestaltet ist zum Abtrennen von in den Saugkanal (12) eingesaugtem Trenngewebe entlang einer Linie, die sich im wesentlichen um eine Längsachse des Saugkanals (12) herum erstreckt.

3. Vorrichtung nach einem der vorangehenden Ansprüche, wobei das Schneidewerkzeug (11) im Saugkanal (12) angeordnet ist und eine distale Kante (17) des Schneidewerkzeugs (11) maximal bis zur Mündung (13) reicht.

4. Vorrichtung nach einem der vorangehenden Ansprüche, wobei das Schneidewerkzeug (11) eine oder mehrere Schneidekanten (17) aufweist, die entlang dem Umfang des Saugkanals (12) an der Innenseite des Saugkanals (12) angeordnet sind.

5. Vorrichtung nach einem der vorangehenden Ansprüche, wobei das Schneidewerkzeug (11) durch ein im Saugkanal (12) angeordnetes Rohrstück gebildet ist.

6. Vorrichtung nach einem der vorangehenden Ansprüche, wobei die Mündung (13) von Rändern umgeben ist, die stumpfkantiger sind als das Schneidewerkzeug (11), und wobei im Bereich der Mündung (13) eine oder mehrere Schulterflächen (20) zum Abstützen des Trenngewebes angeordnet sind.

7. Vorrichtung nach einem der vorangehenden Ansprüche, wobei das Schneidewerkzeug (11) ausgestaltet ist, um Trenngewebe entlang einer sich um eine Längsachse des Saugkanals (12) erstreckenden Linie abzutrennen, wobei die Linie vorzugsweise im wesentlichen kreisförmig ist.

8. Vorrichtung nach einem der vorangehenden Ansprüche, wobei die Mündung (13) schräg zu einer Längsachse der Sonde (8) liegt.

9. Vorrichtung nach einem der vorangehenden Ansprüche, wobei ein distales Ende der Sonde (8) aus einem deformierbaren Material besteht.

10. Vorrichtung nach Anspruch 9, wobei am distalen Ende der Sonde (8) ein Abstützvorrichtung (22) angeordnet ist, welche aus deformierbareren Material als die Sonde (8) besteht.

11. Vorrichtung nach einem der vorangehenden Ansprüche, mit Mitteln zum Erzeugen einer Vibration der Sonde und/oder des Schneidewerkzeugs.

12. Vorrichtung nach einem der vorangehenden Ansprüche, wobei die Mündung einen Durchmesser zwischen 50 µm und 200 µm aufweist.

13. Vorrichtung nach einem der vorangehenden Ansprüche, weiter umfassend eine Absaugvorrichtung (7) zum Erzeugen von Saugpulsen zum Einsaugen von Trenngewebe.

14. Vorrichtung nach Anspruch 13, wobei die Saugpulse höchstens 200 ms lang sind.

15. Vorrichtung nach einem der Ansprüche 13 oder 14, wobei die Absaugvorrichtung ausgestaltet ist um Saugpulse einer ersten, höheren Saugleistung und ein dauerendes Saugen bei einer zweiten, tieferen Saugleistung zu erzeugen.

## Claims

1. Device for treating glaucoma by creating openings between the anterior aqueous chamber and Schlemm's canal of an eye, with a probe (8) which is adapted for insertion in the anterior aqueous chamber such that a distal end (10) of the probe (8) is guidable to a dividing tissue between the anterior aqueous chamber and Schlemm's canal, wherein at the end of the probe (8) a cutting tool (11) for cutting the dividing tissue is arranged, and wherein a suction channel (12) extends through the probe (8) which ends in a orifice (13) being frontally arranged at the distal end of the probe (8), **characterized in that** the cutting tool (11) is arranged in the suction channel (12) at the orifice (13) such that the dividing tissue sucked into the suction channel (12) is removable by the cutting tool (11), wherein a cutting edge (17) of the cutting tool (11) is arranged in a stationary manner in the suction channel (12) maximally to the orifice (13) and wherein the cutting edge (17) is positioned such that dividing tissue for cutting is drawable to the cutting edge (17) by low-pressure in the suction channel (12).

2. Device according to claim 1, **characterized in that** the cutting tool (11) is adapted for cutting off the dividing tissue sucked into the suction channel (12) along a line which essentially extends around a longitudinal axis of the suction channel (12).

3. Device of anyone of the preceding claims, wherein the cutting tool (11) is arranged in the suction channel (12) and a distal edge (17) of the cutting tool (11) extends maximally to the orifice (13).

4. Device of anyone of the preceding claims, wherein the cutting tool (11) comprises one or several cutting edges (17) which are arranged along the circumference of the suction channel (12) in the inside of the suction channel (12).

5. Device of anyone of the preceding claims, wherein the cutting tool (11) is formed by a tube section arranged in the suction channel (12).

6. Device of anyone of the preceding claims, wherein the orifice (13) is surrounded by edges which are more blunt than the separation means (11) and wherein one or more shoulder surfaces (20) are arranged in the area of the orifice (13) for supporting the dividing tissue.

7. Device of anyone of the preceding claims, wherein the cutting tool (11) is adapted for cutting off dividing tissue along a line extending about a longitudinal axis of the suction channel (12) wherein the line is preferably essentially circular.

8. Device anyone of the preceding claims, wherein the orifice (13) is inclined to a longitudinal axis of the probe (8) .

9. Device anyone of the preceding claims, wherein a distal end of the probe (8) is made of a deformable material.

10. Device of claim 9, wherein a support device (22) is arranged at the distal end of the probe (8) which is made of a material that is more deformable than the probe (8).

11. Device of anyone of the preceding claims with means for the generation of a vibration of the probe and/or the cutting tool.

12. Device of anyone of the preceding claims, wherein the orifice has a diameter between 50 µm to 200 µm.

13. Device of anyone of the preceding claims further comprising a suction device (7) for the generation of suction pulses for the aspiration of dividing tissue.

14. Device of claim 13, wherein the suction pulses last maximally 200 ms.

15. Device of claim 13 or 14, wherein the suction device is adapted for the generation of suction pulses of a first higher suction capacity and a permanent suction at a second lower suction capacity.

## Revendications

1. Dispositif pour traiter des glaucomes en générant des ouvertures entre la chambre avant et le canal de Schlemm d'un oeil, avec une sonde (8), qui est appropriée pour s'introduire dans la chambre avant de telle sorte qu'une extrémité (10) distale de la sonde (8) peut être guidée vers un tissu de séparation entre la chambre avant et le canal de Schlemm, un outil de coupe (11) destiné au sectionnement du tissu de séparation étant disposé sur l'extrémité de la sonde (8), et un canal d'aspiration (12) s'étendant à travers la sonde (8), lequel canal débouche dans un débouché (13) côté avant sur l'extrémité distale de la sonde (8) **caractérisé en ce que** l'outil de coupe (11) est disposé dans le canal d'aspiration (12) près du débouché (13), de telle sorte que, du tissu de séparation introduit par aspiration dans le canal d'aspiration (12) peut être sectionné avec l'outil de coupe (11), une arête de coupe (17) de l'outil de coupe (11) étant disposée de façon fixe dans le canal d'aspiration (12) au maximum jusqu'au débouché (13), et l'arête de coupe (17) étant positionnée de telle sorte que, par dépression dans le canal d'aspiration (12), du tissu de séparation peut être tiré vers l'arête de coupe (17) pour la coupe.

2. Dispositif selon la revendication 1, **caractérisé en ce que** l'outil de coupe (11) est conçu pour le sectionnement de tissu de séparation introduit dans le canal d'aspiration (12) par aspiration le long d'une ligne qui s'étend sensiblement autour d'un axe longitudinal du canal d'aspiration (12).

3. Dispositif selon l'une quelconque des revendications précédentes, l'outil de coupe (11) étant disposé dans le canal d'aspiration (12) et une arête (17) distale de l'outil de coupe (11) s'étendant au maximum jusqu'au débouché (13).

4. Dispositif selon l'une quelconque des revendications précédentes, l'outil de coupe (11) présentant une ou plusieurs arêtes de coupe (17), qui sont disposées le long du pourtour du canal d'aspiration (12) sur le côté intérieur du canal d'aspiration (12).

5. Dispositif selon l'une quelconque des revendications précédentes, l'outil de coupe (11) étant formé par une pièce tubulaire disposée dans le canal d'aspiration (12).

6. Dispositif selon l'une quelconque des revendications précédentes, le débouché (13) étant entouré de bords qui présentent une arête plus émoussée que l'outil de coupe (11), et une ou plusieurs surfaces d'épaulement (20) destinées à soutenir le tissu de séparation étant disposées dans la zone du débouché (13).

7. Dispositif selon l'une quelconque des revendications précédentes, l'outil de coupe (11) étant conçu pour sectionner du tissu de séparation le long d'une ligne s'étendant autour d'un axe longitudinal du canal d'aspiration (12), la ligne étant de préférence sensiblement circulaire.

8. Dispositif selon l'une quelconque des revendications précédentes, le débouché (13) étant situé en biais par rapport à un axe longitudinal de la sonde (8).

9. Dispositif selon l'une quelconque des revendications précédentes, une extrémité distale de la sonde (8) étant à base d'un matériau déformable.

10. Dispositif selon la revendication 9, un dispositif de soutien (22) étant disposé sur l'extrémité distale de la sonde (8), lequel dispositif est à base de matériau plus déformable que la sonde (8).

11. Dispositif selon l'une quelconque des revendications précédentes, avec des moyens pour générer une vibration de la sonde et/ou de l'outil de coupe.

12. Dispositif selon l'une quelconque des revendications précédentes, le débouché présentant un diamètre compris entre 50 µm et 200 µm.

13. Dispositif selon l'une quelconque des revendications précédentes, comprenant également un dispositif d'aspiration (7) destiné à générer des impulsions d'aspiration pour aspirer du tissu de séparation.

14. Dispositif selon la revendication 13, les impulsions d'aspiration présentant une longueur maximale de 200 ms.

15. Dispositif selon l'une quelconque des revendications 13 ou 14, le dispositif d'aspiration étant conçu pour générer des impulsions d'aspiration d'une première puissance d'aspiration plus élevée et une aspiration durable avec une seconde puissance d'aspiration plus faible.
